# EUROPEAN PATENT APPLICATION

(11) **EP 4 169 933 A1**
(43) Date of publication of application: **26.04.2023**
(21) Application number: 21828185.5
(22) Date of filing: 23.06.2021
(51) Int. Cl.: C07K 1/00, C09K 3/00, C12M 1/40, A23L 33/00

(54) **METHOD OF INACTIVATING ALLERGEN AND ALLERGEN INACTIVATION DEVICE**

(30) Priority: 23.06.2020 JP 2020108223; 13.01.2021 JP 2021003786
(71) Applicant: Panasonic Intellectual Property Management Co., Ltd., Osaka-shi, Osaka 540-6207 (JP)
(72) Inventor: HATSUGAI, Noriyuki, Osaka-shi, Osaka 540-6207 (JP); OKUMURA, Yasuaki, Osaka-shi, Osaka 540-6207 (JP); WAYAMA, Fumiya, Osaka-shi, Osaka 540-6207 (JP)
(74) Representative: Novagraaf International SA
(86) International application number: PCT/JP2021/023754
(87) International publication number: WO 2021/261511

(57) **Abstract**

An allergen inactivation method includes an inactivation step (S104) of inactivating an allergen present in a reaction system by reduction via a reduced redox protein, and a reduction process (S103) of reducing an oxidized redox protein produced by oxidation of the reduced redox protein in the inactivating to the reduced redox protein by donating an electron from an electrode connected to an external power supply outside the reaction system to the oxidized redox protein.

## Description

### [Technical Field]

The present disclosure relates to an allergen inactivation method and an allergen inactivation device that inactivate allergens by reducing disulfide bonds in allergen proteins.

### [Background Art]

Allergies have become a serious social problem with the number of sufferers increasing each year, especially in developed countries. Proteins with disulfide bonds are known to be components that induce allergies (hereafter also referred to as allergens). Because disulfide bonds are very strong bonds, the conformational structure of proteins with disulfide bonds is not easily unraveled. Proteins with disulfide bonds are therefore difficult to digest in digestive organs such as the stomach. Thus, proteins with disulfide bonds are more likely to cause allergies (in other words, they are more allergenic).

For example, Patent Literature (PTL) 1 discloses a method of decreasing the allergenicity of allergens by cleaving the disulfide bonds of allergens using thioredoxin, which is a low molecular weight redox protein.

### [Citation List]

### [Patent Literature]

[PTL 1] Japanese Unexamined Patent Application Publication (Translation of PCT Application) No. 2001-520027

### [Summary of Invention]

### [Technical Problem]

However, the method described in PTL 1 requires adding an excess amount of thioredoxin to the sample relative to the amount of allergens in order to cleave the disulfide bonds of all allergens in the sample. Consequently, the method disclosed in PTL 1 of inactivating allergens in the sample can hardly be called efficient.

In view of this, the present disclosure provides an allergen inactivation method and an allergen inactivation device that can inactivate allergens efficiently.

### [Solution to Problem]

An allergen inactivation method according to one aspect of the present disclosure includes: inactivating an allergen present in a reaction system by reduction via a reduced redox protein; and reducing an oxidized redox protein produced by oxidation of the reduced redox protein in the first process to the reduced redox protein by donating an electron from an electrode connected to an external power supply outside the reaction system to the oxidized redox protein.

An allergen inactivation device according to one aspect of the present disclosure includes: an electrode for donating an electron to a redox protein that inactivates an allergen by reduction by application of voltage; a power supply that applies voltage to the electrode; and a controller that controls application of voltage by the power supply.

### [Advantageous Effects of Invention]

According to the present disclosure, it is possible to provide an allergen inactivation method and an allergen inactivation device that can inactivate allergens efficiently.

### [Brief Description of Drawings]

[FIG. 1]

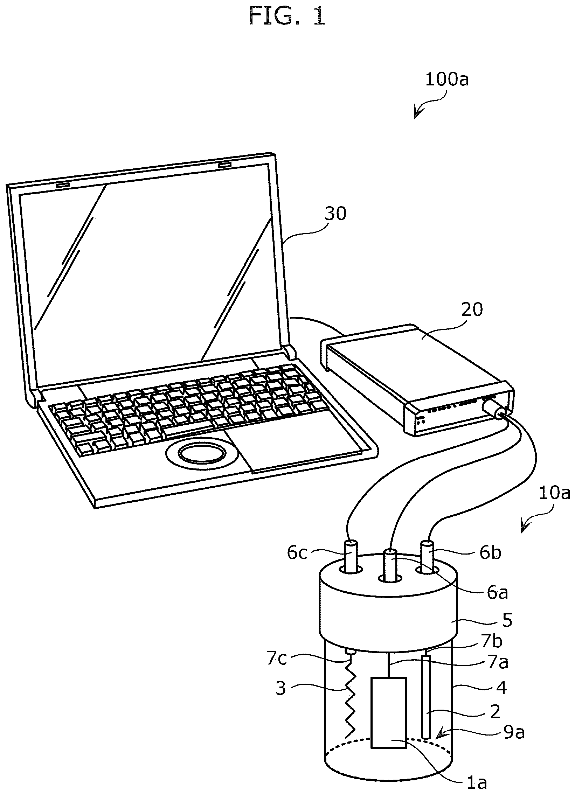

   FIG. 1 illustrates one example of the configuration of an allergen inactivation device according to Embodiment 1.
[FIG. 2]

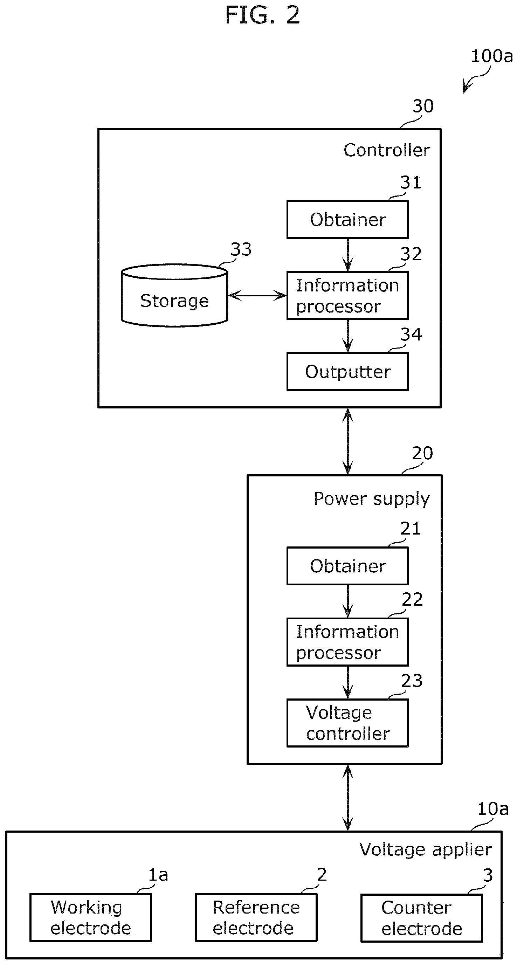

   FIG. 2 is a block diagram illustrating one example of the functional configuration of the allergen inactivation device according to Embodiment 1.
[FIG. 3A]

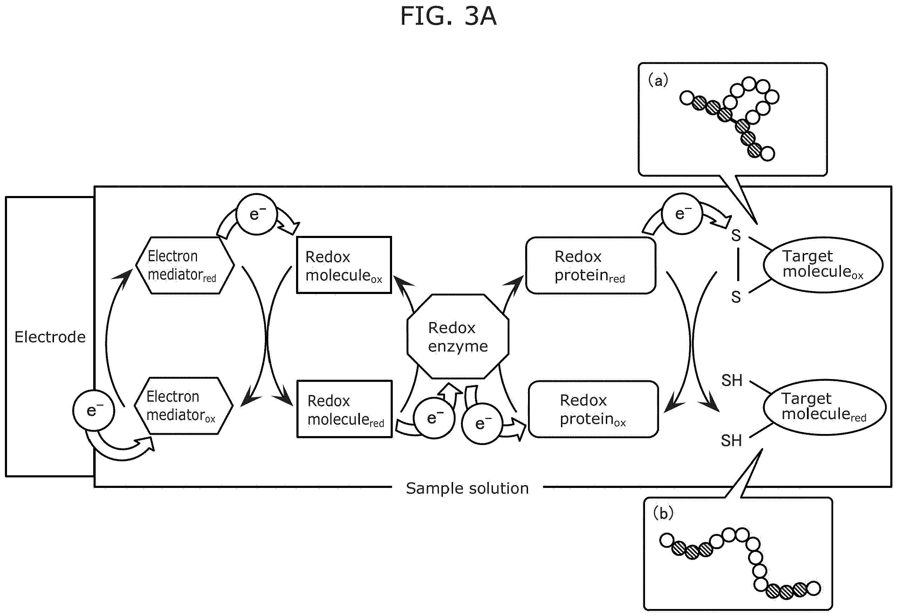

   FIG. 3A is a first schematic diagram illustrating the components in a sample solution and the electron transfer reactions between them.
[FIG. 3B]

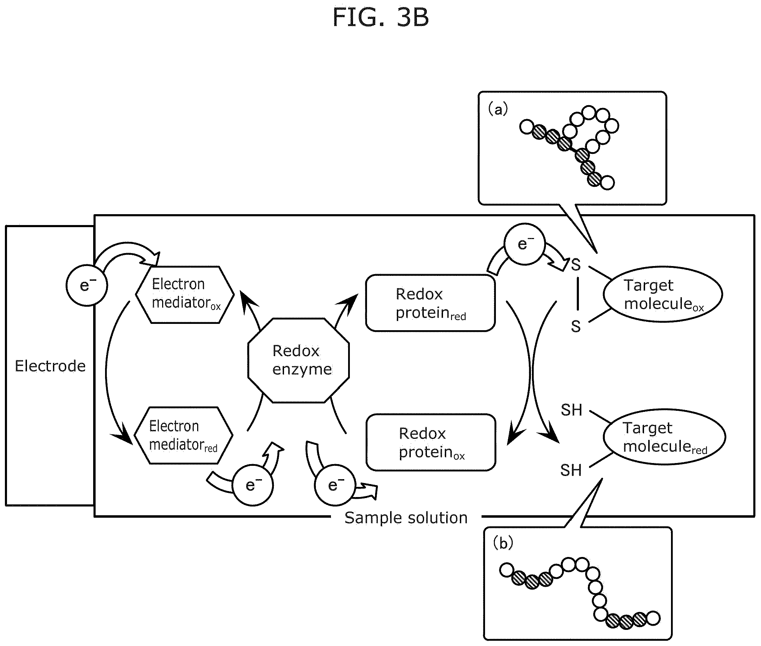

   FIG. 3B is a second schematic diagram illustrating the components in a sample solution and the electron transfer reactions between them.

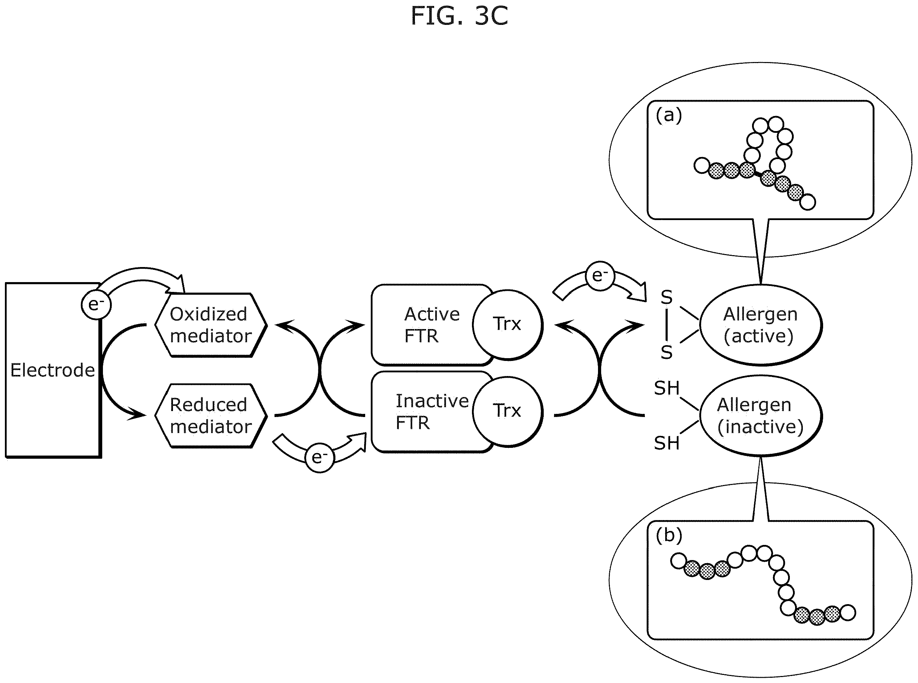

[FIG. 3C] FIG. 3C illustrates an application example of a fusion protein.
[FIG. 4]

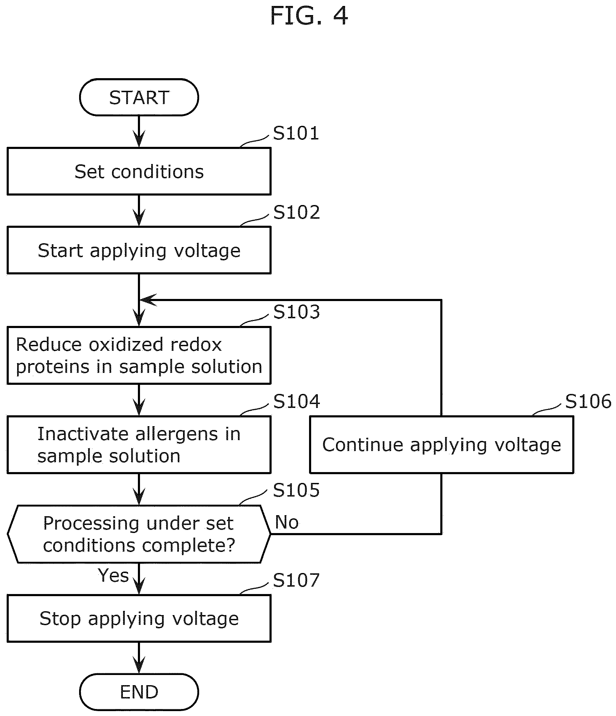

   FIG. 4 is a flowchart illustrating one example of the operation of the allergen inactivation device according to Embodiment 1.
[FIG. 5]

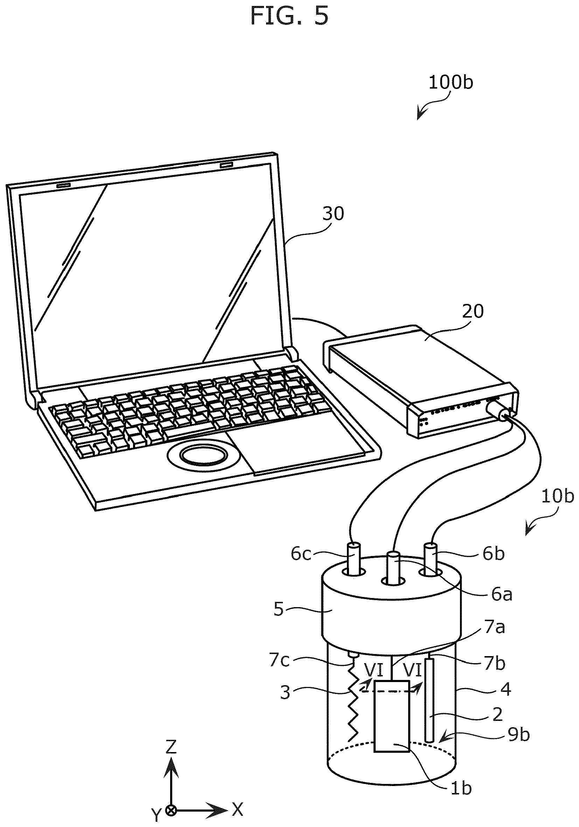

   FIG. 5 illustrates one example of the configuration of an allergen inactivation device according to Embodiment 2.
[FIG. 6]

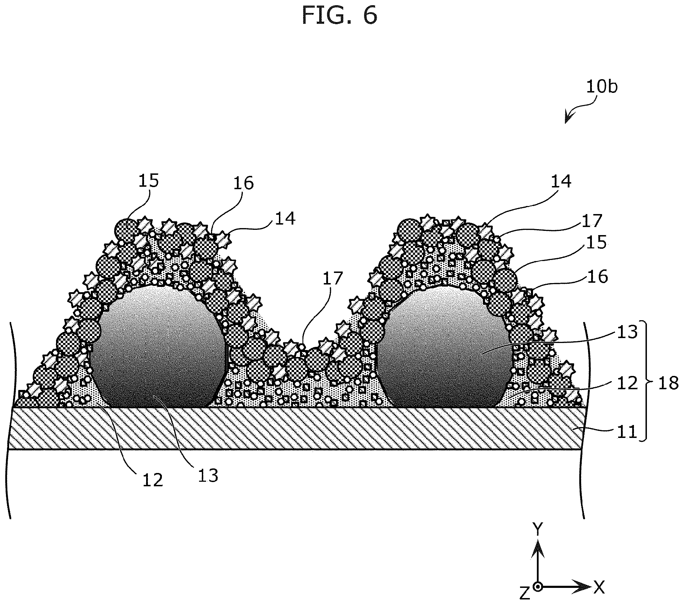

   FIG. 6 is a schematic cross-sectional view taken at line VI-VI of the working electrode illustrated in FIG. 5.
[FIG. 7]

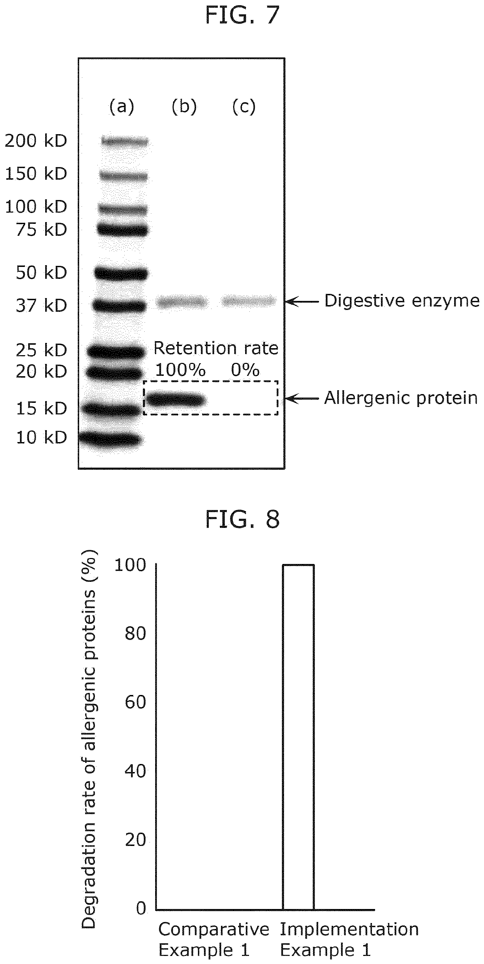

   FIG. 7 illustrates electrophoresis images after SDS-PAGE in Comparative Example 1 and Implementation Example 1.
[FIG. 8]
   FIG. 8 is a graph illustrating the degradation rate of allergenic proteins after treatment with digestive enzymes in Comparative Example 1 and Implementation Example 1.
[FIG. 9]

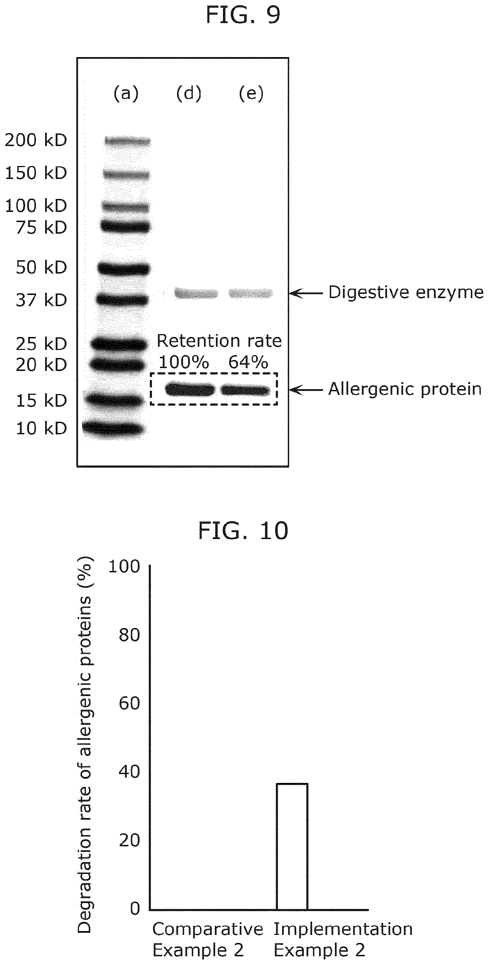

   FIG. 9 illustrates electrophoresis images after SDS-PAGE in Comparative Example 2 and Implementation Example 2.
[FIG. 10]
   FIG. 10 is a graph illustrating the degradation rate of allergenic proteins after treatment with digestive enzymes in Comparative Example 2 and Implementation Example 2.
[FIG. 11]

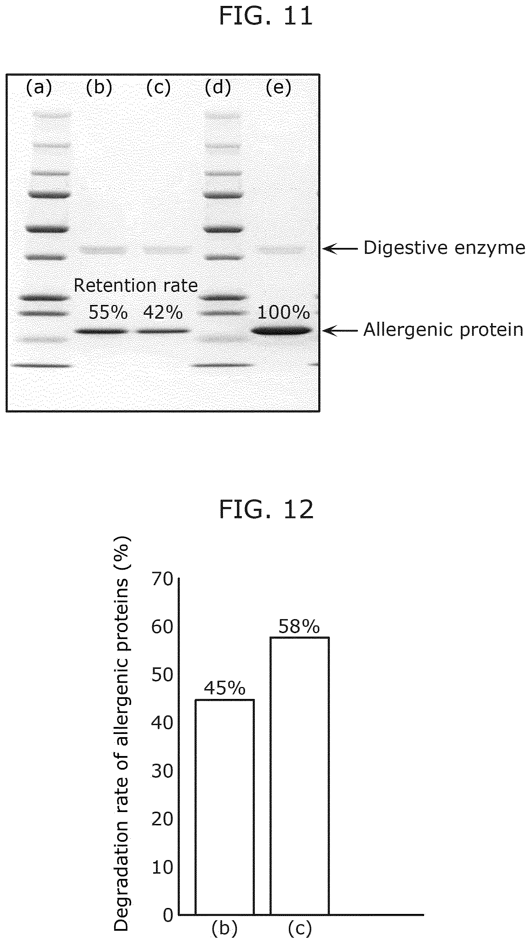

   FIG. 11 illustrates electrophoresis images after SDS-PAGE in Implementation Example 3 and Implementation Example 4.
[FIG. 12]
   FIG. 12 is a graph illustrating the degradation rate of allergenic proteins after treatment with digestive enzymes in Implementation Example 3 and Implementation Example 4.

### [Description of Embodiments]

### Knowledge Leading to Present Disclosure

In recent years, the number of allergy sufferers has increased and become a serious social problem. As noted in the Background Art section, allergens are components that cause allergies. For example, the majority of food allergens are proteins (also called allergenic proteins) found in food. Proteins have a conformational structure in which amino acids are linked in chains and folded into a helical or sheet-like shape. Food allergy symptoms are caused, for example, by IgE antibodies, which bind to a specific food allergen, binding to a portion of the protein consisting of a specific amino acid sequence in its conformational structure. Reducing the binding of IgE antibodies to a specific food allergen can therefore reduce the allergenicity of the allergen. Methods to reduce the allergenicity of allergens include, for example, denaturing the protein by adding heat, acid, or enzymes. When a protein is denatured, the protein's conformational structure is unraveled and/or the amino acid sequence of the protein is cleaved. As a result, the portion of the protein that binds to the IgE antibody is deformed. However, disulfide bonds are very strong bonds that are not easily cleaved by heat, acids, or enzymes (for example, gastric digestive enzymes). Therefore, the conformational structure of proteins with disulfide bonds is difficult to unravel. Proteins with disulfide bonds are thus said to be more likely to cause allergies (i.e., more allergenic) if the disulfide bonds are present in the portion that binds to IgE antibodies, because the portion that binds to IgE antibodies is more likely to be retained.

There is therefore a need for an allergen weakening technique that efficiently cleaves the disulfide bonds that allergens possess. For example, PTL 1 discloses a method of decreasing the allergenicity of allergens by cleaving the disulfide bonds of allergenic proteins using thioredoxin, which is a low molecular weight redox protein.

However, with the method described in PTL 1, thioredoxin loses its reducing power when it cleaves the disulfide bonds of allergens. More specifically, thioredoxin (also called reduced thioredoxin) reduces disulfide bonds to thiol groups when it itself is oxidized. This cleaves the disulfide bonds. The reduced thioredoxin loses its reducing power that allows it to reduce the disulfide bonds because it is converted to oxidized thioredoxin (also called inactive thioredoxin). The method described in PTL 1 therefore requires adding an excess amount of thioredoxin to the sample in order to cleave the disulfide bonds of all allergens in the sample. Consequently, the method described in PTL 1 of reducing allergens (i.e., inactivating allergens) can hardly be called efficient.

For example, with the method described in PTL 1, if the above sample is food, adding an excessive amount of thioredoxin to the food may cause a large amount of unreacted thioredoxin to remain in the food, which may alter other components in the food. Application of the method described in PTL 1 to food products is therefore not appropriate from the standpoint of quality and consumer safety. PTL 1 neither mentions nor implies that inactive thioredoxin can be activated for continuous use.

In view of the above, the inventors of the present application worked diligently to overcome the above problems and discovered a method to repeatedly activate redox proteins that have lost their reducing power by reducing allergens. The inventors found that this makes it possible to efficiently reduce allergens using a smaller amount of redox protein than is needed to reduce all allergens in the sample.

According to the present disclosure, it is possible to provide an allergen inactivation method and an allergen inactivation device that can inactivate allergens efficiently.

### One Aspect of the Present Disclosure

Hereinafter, an overview of one aspect of the present disclosure will be given.

An allergen inactivation method according to one aspect of the present disclosure includes: inactivating an allergen present in a reaction system by reduction via a reduced redox protein; and reducing an oxidized redox protein produced by oxidation of the reduced redox protein in the inactivating to the reduced redox protein by donating an electron from an electrode connected to an external power supply outside the reaction system to the oxidized redox protein.

With this, since oxidized redox proteins can be reduced to reduced redox proteins, redox proteins that once lost their activity can be reactivated and reused to inactivate allergens. Therefore, a small amount of redox protein relative to the amount of allergens can be used to reduce the allergens in the reaction system. The allergen inactivation method can therefore efficiently inactivate allergens.

In the allergen inactivation method according to one aspect of the present disclosure, in the reducing, an electron may be donated from the electrode to a redox enzyme, and an electron may be donated from the redox enzyme to the oxidized redox protein.

With this, the transfer rate and the amount of energy of electrons donated from the electrode to the oxidized redox proteins can be adjusted depending on the combination of the redox enzyme and the oxidized redox protein used. The allergen inactivation method can therefore improve the efficiency of the electron transfer reaction between the electrode and the redox proteins.

In the allergen inactivation method according to one aspect of the present disclosure, in the reducing, an electron may be donated from the electrode to a redox molecule, an electron may be donated from the redox molecule to a redox enzyme, and an electron may be donated from the redox enzyme to the oxidized redox protein.

With this, the transfer rate and the amount of energy of electrons donated from the electrode to the oxidized redox proteins can be adjusted depending on the combination of the redox molecule, the redox enzyme, and the oxidized redox protein used. The allergen inactivation method can therefore improve the efficiency of the electron transfer reaction between the electrode and the redox proteins.

In the allergen inactivation method according to one aspect of the present disclosure, in the reducing, an electron may be donated from the electrode to an electron mediator, an electron may be donated from the electrode mediator to a redox molecule, an electron may be donated from the redox molecule to a redox enzyme, and an electron may be donated from the redox enzyme to the oxidized redox protein.

With this, the transfer rate and the amount of energy of electrons donated from the electrode to the oxidized redox proteins can be adjusted depending on the combination of the electron mediator, the redox molecule, the redox enzyme, and the oxidized redox protein used. The allergen inactivation method can therefore improve the efficiency of the electron transfer reaction between the electrode and the redox proteins.

In the allergen inactivation method according to one aspect of the present disclosure, the allergen may include a disulfide bond.

With this, the disulfide bonds of the allergens are reduced to thiol groups by the reduced redox proteins. This cleaves the disulfide bonds of the allergens. The allergen inactivation method can therefore cleave the disulfide bonds of the allergens.

In the allergen inactivation method according to one aspect of the present disclosure, the redox protein may be thioredoxin, glutathione, a protein with at least one thioredoxin-like domain, or a protein with at least one glutathione-like motif.

With this, the redox protein can reduce disulfide bonds because it includes a cysteine-derived thiol group. The allergen inactivation method can therefore inactivate allergens by reducing the disulfide bonds of the allergens.

In the allergen inactivation method according to one aspect of the present disclosure, the redox enzyme may be: (i) an enzyme that catalyzes reduction of oxidized thioredoxin, including a NADPH-thioredoxin reductase or a ferredoxin-thioredoxin reductase; or (ii) an enzyme that catalyzes reduction of oxidized glutathione, including a glutathione reductase.

With this, when the redox protein is thioredoxin or glutathione, the redox enzymes can efficiently reduce the oxidized redox proteins produced by reducing the disulfide bonds to the reduced redox proteins. The allergen inactivation method can therefore efficiently inactivate allergens because redox proteins that have lost their reducing power can be activated (i.e., reduced) with a small amount of redox protein.

In the allergen inactivation method according to one aspect of the present disclosure, the redox molecule may be nicotinamide adenine dinucleotide phosphate or ferredoxin.

With this, the redox molecules can donate electrons to the redox enzymes efficiently, thus efficiently reducing the redox enzymes. The redox enzymes can therefore efficiently reduce oxidized redox proteins. The allergen inactivation method can therefore efficiently inactivate allergens because redox proteins that have lost their reducing power can be efficiently activated (i.e., reduced) with a small amount of redox protein.

In the allergen inactivation method according to one aspect of the present disclosure, the electron mediator may be a compound with a bipyridine skeleton.

With this, since the electron mediator has multiple nitrogen-containing heterocyclic rings, the electrons necessary for the redox protein reduction are efficiently donated to the redox molecule due to the multiple contributions of the nitrogen contained in the nitrogen-containing heterocyclic rings. The allergen inactivation method can therefore efficiently donate electrons donated from the electrode via the electron mediators to the oxidized redox proteins. With this, since oxidized redox protein can be efficiently activated (i.e., reduced), the allergens can be efficiently inactivated with a small amount of redox protein. The allergen inactivation method can therefore efficiently inactivate the allergens because the oxidized redox proteins can be efficiently reduced to reduced redox proteins with a small amount of redox protein.

In the allergen inactivation method according to one aspect of the present disclosure, a reaction temperature in the reaction system may be greater than or equal to 4 °C and less than 60 °C.

With this, the reduced redox proteins can reduce allergens in an environment greater than or equal to 4 °C and less than 60 °C. The allergen inactivation method can therefore inactivate allergens in an environment greater than or equal to 4 °C and less than 60 °C.

In the allergen inactivation method according to one aspect of the present disclosure, a voltage applied to the electrode by the external power supply may be between -1.0 V and 0 V, inclusive.

With this, since the applied voltage is adjusted so that the voltage applied to the electrode by the external power supply is within the range of -0.1 V to 0 V, inclusive, the allergen inactivation method can adjust the efficiency of the electron transfer reaction according to the components in the reaction system.

An allergen inactivation device according to one aspect of the present disclosure includes: an electrode for donating an electron to a redox protein that inactivates an allergen by reduction by application of voltage; a power supply that applies voltage to the electrode; and a controller that controls application of voltage by the power supply.

With this, the allergen inactivation device can donate electrons from the electrode to redox proteins in oxidized form (i.e., oxidized redox proteins), which are produced by oxidizing the allergens by reduction, to reduce them to redox proteins in reduced form (i.e., reduced redox proteins). The allergen inactivation device can therefore activate redox proteins that once lost their activity due to redox reactions with allergens and reuse them to reduce allergens. Accordingly, the allergen inactivation device can efficiently inactivate allergens since it can reduce allergens using a small amount of redox protein relative to the amount of allergens.

In the allergen inactivation device according to one aspect of the present disclosure, for example, the redox protein may be immobilized on the electrode.

This eliminates the need to add redox proteins to the allergen-containing sample for allergen inactivation. The allergen inactivation device can therefore easily reduce allergens and thus efficiently inactivate allergens. The allergen inactivation device can prevent redox proteins from being mixed into the allergen-containing sample.

In the allergen inactivation device according to one aspect of the present disclosure, for example, a redox enzyme that donates an electron to the redox protein may further be immobilized on the electrode.

This eliminates the need to further add redox enzymes to the allergen-containing sample for allergen inactivation. The allergen inactivation device can therefore more easily reduce allergens and thus efficiently inactivate allergens. The allergen inactivation device can prevent redox enzymes from further being mixed into the allergen-containing sample.

In the allergen inactivation device according to one aspect of the present disclosure, for example, a redox molecule that donates an electron to the redox enzyme may further be immobilized on the electrode.

This eliminates the need to further add redox molecules to the allergen-containing sample for allergen inactivation. The allergen inactivation device can therefore more easily reduce allergens and thus efficiently inactivate allergens. The allergen inactivation device can prevent redox molecules from further being mixed into the allergen-containing sample.

In the allergen inactivation device according to one aspect of the present disclosure, for example, an electron mediator that donates an electron to the redox molecule may further be immobilized on the electrode.

This eliminates the need to further add electron mediators to the allergen-containing sample for allergen inactivation. The allergen inactivation device can therefore more easily reduce allergens and thus efficiently inactivate allergens. The allergen inactivation device can prevent electron mediators from further being mixed into the allergen-containing sample.

In the allergen inactivation device according to one aspect of the present disclosure, for example, an electron mediator that mediates electron transfer between the electrode and the redox protein may be immobilized on the electrode.

With this, the allergen inactivation device can efficiently reduce allergens with a small amount of redox protein because of the increased efficiency of the electron transfer reaction between the electrode and the redox proteins. The allergen inactivation device can therefore efficiently inactivate allergens.

General or specific aspects of the present disclosure may be realized as a system, a method, a device, an integrated circuit, a computer program, a computer readable medium such as a CD-ROM, or any given combination thereof.

Hereinafter, embodiments are specifically described with reference to the drawings.

Each of the embodiments described below shows a general or specific example of the present disclosure. The numerical values, shapes, materials, elements, the arrangement and connection of the elements, steps, the order of the steps, etc., indicated in the following embodiments are mere examples, and therefore do not intend to limit the claims. Therefore, among elements in the following embodiments, those not recited in any of the broadest, independent claims are described as optional elements. The figures are schematic illustrations and are not necessarily precise depictions. Elements that are essentially the same have the same reference signs in the figures, and duplicate description may be omitted or simplified.

The mutually orthogonal X-axis, Y-axis, and Z-axis directions illustrated in the figures will be used as appropriate in the description. In particular, the positive side in the Z-axis direction may be described as the upper side, and the negative side in the Z-axis direction may be described as the lower side.

In the present disclosure, terms indicating relationships between elements such as "parallel" and "perpendicular", terms indicating shapes of elements such as "rectangular", and numerical ranges refer not only to their strict meanings, but encompass a range of essentially equivalents, such as a range of deviations of a few percent.

In the figures of the present disclosure, dashed lines indicate the boundaries of what is not visible from the surface, as well as regions.

### [Embodiment 1]

Hereinafter, Embodiment 1 will be described in detail with reference to FIG. 1 through FIG. 4.

### Allergen Inactivation Device

### 1. Overview

First, an overview of the allergen inactivation device according to Embodiment 1 will described with reference to FIG. 1. FIG. 1 illustrates one example of the configuration of allergen inactivation device 100a according to Embodiment 1.

Allergen inactivation device 100a is a device that continuously inactivates allergens by repeatedly activating redox proteins that have lost their reducing power, by donating electrons to the redox proteins that inactivate the allergens by reduction. Allergen inactivation device 100a can adjust the transfer rate of electrons and the amount of energy donated from the electrode (working electrode 1a) to the redox proteins by controlling the voltage applied to the electrode (working electrode 1a) by power supply 20.

As mentioned above, allergens are components that induce allergies. Allergens have sites (specific binding sites) that specifically bind to antibodies (for example, IgE antibodies) of people with allergic diseases. Inactivating an allergen means reducing the allergenicity of the allergen, for example, by changing the structure (for example, the amino acid sequence) of the specific binding site of the allergen so that IgE antibodies are less likely to recognize the specific binding site of the allergen.

For example, the allergen targeted for inactivation in Embodiment 1 has a disulfide bond. Allergens with disulfide bonds are, for example, allergenic proteins. As mentioned above, disulfide bonds are very strong bonds that are not easily broken by heat, acids, or enzymes (for example, gastric digestive enzymes). Allergens with disulfide bonds are therefore more likely to cause allergies. If an allergen has a disulfide bond, reducing the allergen means reducing the disulfide bond of the allergen. Allergen reduction will be described in greater detail later.

### 2. Configuration

Next, the configuration of allergen inactivation device 100a according to Embodiment 1 will be described with reference to FIG. 1 and FIG. 2. FIG. 2 is a block diagram illustrating one example of the functional configuration of allergen inactivation device 100a according to Embodiment 1.

Allergen inactivation device 100a according to Embodiment 1 includes an electrode (working electrode 1a) for donating electrons to redox proteins that inactivate allergens by reduction through the application of voltage, power supply 20 for applying voltage to the electrode (working electrode 1a), and controller 30 for controlling the application of voltage by power supply 20. The electrode that donates electrons to the redox proteins (hereinafter also simply referred to as working electrode 1a) is a component of voltage applier 10a.

### Voltage Applier

Voltage applier 10a donates electrons from the electrode (working electrode 1a) to the redox proteins. Voltage applier 10a is, for example, a three-electrode cell that includes working electrode 1a, reference electrode 2, counter electrode 3, cell 4, lid 5, terminals 6a, 6b, and 6c, and leads 7a, 7b, and 7c. Voltage applier 10a may be a two-electrode cell that includes, for example, working electrode 1a and counter electrode 3.

Working electrode 1a is an electrode that is sensitive to electrochemical responses to trace components in sample solution 9a at the electrode surface thereof. Counter electrode 3 is an electrode that establishes a potential difference with working electrode 1a. Working electrode 1a and counter electrode 3 are made of a conductive material. The conductive material may be, for example, a carbon material, a conductive polymer material, a semiconductor, or a metal. Carbon material examples include carbon nanotube, Ketjen black (registered trademark), glassy carbon, graphene, fullerene, carbon fiber, carbon fabric, and carbon aerogel. Conductive polymer material examples include polyaniline, polyacetylene, polypyrrole, poly(3,4-ethylenedioxythiophene), poly(p-phenylenevinylene), polythiophene, and poly(p-phenylene sulfide). Semiconductor examples include silicone, germanium, indium tin oxide (ITO), titanium oxide, copper oxide, and silver oxide. Metal examples include gold, platinum, silver, titanium, aluminum, tungsten, copper, iron, and palladium. Here, working electrode 1a is, for example, a glassy carbon electrode, and counter electrode 3 is a platinum electrode. The conductive material is not particularly limited as long as the conductive material is not decomposed by its own oxidation reaction.

Reference electrode 2 is an electrode that does not react with the components in sample solution 9a and maintains a constant potential, and is used to control the potential difference between working electrode 1a and reference electrode 2 to a constant level by power supply 20. Here, reference electrode 2 is a silver/silver chloride electrode.

Cell 4 is a holder for holding sample solution 9a in which allergens are present. Sample solution 9a contains at least allergens and redox proteins that reduce the allergens. In addition to allergens and redox proteins, sample solution 9a may also contain redox enzymes that reduce the redox proteins. In addition to allergens, redox proteins, and redox enzymes, sample solution 9a may also contain redox molecules that reduce the redox enzymes. In addition to allergens, redox proteins, redox enzymes, and redox molecules, sample solution 9a may also contain electron mediators that are involved in the electron transfer between the electrode and the redox proteins. Hereinafter, an example in which sample solution 9a contains allergens, redox proteins, redox enzymes, redox molecules, and electron mediators will be described. As used herein, reducing allergens means reducing the disulfide bonds of allergens.

Next, the components in sample solution 9a will be described with reference to FIG. 3A. FIG. 3A is a first schematic diagram illustrating the components in sample solution 9a and the electron transfer reactions between them (electrons are denoted as "e⁻" in the figure). An electron transfer reaction is a reaction involving the transfer of electrons, and is also referred to as a redox reaction. Each component present in the reaction system is reduced when it receives an electron and is oxidized when it donates an electron. Therefore, each component has two forms, oxidized (ox) and reduced (red).

For example, as illustrated in FIG. 3A, the allergen (target moleculeₒₓ in the figure) has a disulfide bond (-S-S-). Allergenic proteins with disulfide bonds (hereinafter simply referred to as allergenic proteins) are allergens derived from, for example: foods such as beans, wheat, milk, seafood, eggs, and rice; the environment such as pollen, animals, nematodes, fungi, molds, and mites; and animals such as animal hair and dander.

The disulfide bond of the allergen (target moleculeₒₓ) is reduced to a thiol group by donation of an electron from a reduced redox protein (redox protein_{red}). Above the target moleculeₒₓ, FIG. 3A schematically illustrates the portion (a) containing the disulfide bond of the allergen before reduction. Below the target molecule_{red}, FIG. 3A schematically illustrates the portion (b) corresponding to (a) above in the allergen after reduction. In (a) and (b), the white circles are amino acids, and the hatched circles indicate the amino acid sequence(s) identified by the IgE antibodies. As illustrated in (a) and (b), when the disulfide bond of the allergen is reduced, the loop portion of the amino acid sequence is unraveled and a different amino acid sequence is inserted in the middle of the amino acid sequence identified by the IgE antibodies. This increases the likelihood that IgE antibodies will be unable to identify specific binding sites, and thus reduces the allergenicity of the allergen.

Note that in (a) and (b) above, the loop portion is given as one non-limiting example. In the conformational structure of a protein, functional sites are formed when amino acid residues that are far apart in the amino acid sequence of the peptide chain of the protein approach each other. The reduction of the disulfide bond breaks the connections between the secondary structures of the protein that were connected by the disulfide bond, and the functional sites formed by the connections between the secondary structures are no longer maintained. More specifically, disulfide bonds are bonds that connect the secondary structures of proteins to each other and strengthen the conformational structure of the protein. Therefore, when the disulfide bond is cleaved, the connection between the secondary structures is broken, increasing the degree of freedom (fluctuation) of the conformational structure of the allergenic protein. As a result, functional sites of allergenic proteins (for example, conformational epitopes) are less likely to be maintained, and IgE antibodies are more likely to be unable to identify specific binding sites of allergenic proteins.

Moreover, as a result of the reduction of the disulfide bond increasing the degree of freedom (fluctuation) of the conformational structure of the allergenic protein, it easier for digestive enzymes to act on the cleavage sites where the peptide chain of the allergenic protein is cleaved by the digestive enzymes, thus making the allergenic protein more easily digestible by the digestive enzymes.

In present disclosure, redox enzymes can also transfer electrons without the use of redox molecules, as illustrated in FIG. 3B. FIG. 3B is a second schematic diagram illustrating the components in sample solution 9a and the electron transfer reactions between them (electrons are denoted as "e⁻" in the figure). The mechanism that enables this is that (i) the shape of the binding site of the redox molecule to the redox enzyme is similar to that of the binding site of the electron mediator, and (ii) when the redox potentials of the redox molecule and the electron mediator are equivalent, the redox enzyme can transfer electrons without the redox molecule. Therefore, it is sufficient so long as the redox enzymes can reduce the redox proteins, but is beneficial when used in combination with electron mediators and redox enzymes that satisfy (i) and (ii) above.

Redox proteins are proteins, polypeptides, or oligopeptides of any size or structure. Redox proteins inactivate allergens by reducing them. For example, redox proteins cleave the disulfide bonds of allergenic proteins by reducing them to thiol groups. As a result, not only does the increased degree of freedom (fluctuation) of the conformational structure of the allergenic protein due to the disulfide bond make it more difficult for IgE antibodies to identify the specific binding site of the allergenic protein, as described above, but it also makes it easier for digestive enzymes to act on the cleavage site, thereby inactivating the allergenicity. The redox protein that reduces a disulfide bond is, for example, thioredoxin, glutathione, a protein with at least one thioredoxin-like domain, or a protein with at least one glutathione-like motif. These redox proteins have thiol groups. For example, thioredoxin is a low molecule weight redox protein with an active site motif having an amino acid sequence consisting of Trp (tryptophan)-Cys (cysteine)-Gly (glycine)-Pro (proline)-Cys (cysteine). Thioredoxin comes in two forms, a reduced form and an oxidized form, depending on the redox state of the thiol groups of the two Cys in the active site. Glutathione is a tripeptide consisting of Glu (glutamic acid)-Cys (cysteine)-Gly (glycine). Glutathione reduces a disulfide bond of an allergenic protein to a thiol group through the reducing power of the thiol group of Cys. Reduced glutathione is a tripeptide consisting of the above three amino acids. Oxidized glutathione is a molecule consisting of two molecules of reduced glutathione joined by a disulfide bond.

A protein with at least one thioredoxin-like domain is, for example, a protein with at least one thioredoxin-like domain containing a Cys-AAc₁-AAc₂-Cys active site. AAc₁ and AAc₂ may be any amino acid residue other than cysteine residue. The number of amino acid residues between the two Cys in the active site is not limited to the two amino acid residues of AAc₁ and AAc₂, and may be three or four, for example. The thioredoxin-like domain may contain at least one Cys-AAc₁-AAc₂-Cys active site. For example, the thioredoxin-like domain may contain a Cys-AAc₁-AAc₂-Cys active site and a Cys-AAc₁'-AAc₂'-Cys active site, and may contain a Cys-AAc₁-AAc₂-Cys-AAc₁'-AAc₂'-Cys active site. AAc₁ and AAc₁' may be the same or different amino acid residues. Additionally, AAc₂ and AAc₂' may be the same or different amino acid residues.

A protein with at least one glutathione-like motif is, for example, a protein with at least one AAc₃-Cys-AAc₄ active site, for example. Stated differently, an AAc₃-Cys-AAc₄ active site is a motif similar to the chemical properties of glutathione (also referred to as a glutathione-like motif). AAc₃ may be the same acidic amino acid as Glu in the glutathione, and AAc₄ may be the same neutral amino acid as Gly in the glutathione. AAc₃ may be, for example, Glu, γ-Glu, Asp (aspartic acid), β-Asp, GluGly, γ-GluGly, AspGly, or β-AspGly. AAc₄ may be, for example, Gly, Phg (phenylglycine), Ala (alanine), β-Ala, or Phe (phenylalanine).

Redox enzymes are enzymes that catalyze the redox reaction of redox proteins. Redox enzymes donate electrons to redox proteins. More specifically, redox enzymes donate electrons donated from working electrode 1a to oxidized redox proteins. Redox enzymes may donate electrons from working electrode 1a via electron mediators, redox proteins, or both electron mediators and redox proteins. The redox enzyme is, for example, (i) an enzyme that catalyzes the reduction of oxidized thioredoxin, including a nicotinamide adenine dinucleotide phosphate (NADPH)-thioredoxin reductase or a ferredoxin-thioredoxin reductase, or (ii) an enzyme that catalyzes the reduction of oxidized glutathione, including a glutathione reductase.

For example, if the redox protein is thioredoxin, the redox enzyme is an enzyme that catalyzes the reduction of oxidized thioredoxin, including NADPH-thioredoxin reductase or ferredoxin-thioredoxin reductase. Enzymes that catalyze the reduction of oxidized thioredoxin are, for example, polypeptides or proteins with thioredoxin reducing activity. Such an enzyme may be, for example, an NADPH-thioredoxin reductase or a ferredoxin-thioredoxin reductase, or a mutant enzyme in which a portion of the amino acid sequence of the NADPH-thioredoxin reductase or the ferredoxin-thioredoxin reductase is mutated. The enzyme may be a metalloenzyme containing metal atoms such as iron, chromium, manganese, magnesium, calcium, cobalt, molybdenum, zinc, copper, or nickel in the active site of the NADPH-thioredoxin reductase or the ferredoxin-thioredoxin reductase. The enzyme may be a hybrid enzyme of a fusion protein in which the NADPH-thioredoxin reductase or the ferredoxin-thioredoxin reductase and the thioredoxin are fused via a linker peptide. In such cases, when the fusion protein accepts electrons donated from the electrode to the electron mediator, it donates electrons to the allergen.

Linker peptides are for fusing the thioredoxin reductase (for example, NADPH-thioredoxin reductase or ferredoxin-thioredoxin reductase) and thioredoxin described above to create a fusion protein. Linker peptides fuse thioredoxin to the thioredoxin reductase so as to allow the redox-active disulfide of thioredoxin to interact with the redox-active disulfide of the thioredoxin reductase.

For example, if the redox protein is glutathione, the redox enzyme is an enzyme that catalyzes the reduction of oxidized glutathione, including glutathione reductase. Enzymes that catalyze the reduction of oxidized glutathione are, for example, polypeptides or proteins with glutathione reducing activity. Such an enzyme may be, for example, a riboflavin-dependent glutathione reductase, such as flavin adenine dinucleotide (FAD) or flavin mononucleotide (FMN), or a NADPH-dependent glutathione reductase.

FIG. 3C illustrates an application example of a fusion protein. FIG. 3C illustrates an example of a fusion protein being applied to inactivate an allergen. In cases in which a fusion protein is applied to inactivate an allergen, when the fusion protein accepts electrons donated from the electrode to the mediator, it donates electrons to the allergen. The disulfide bond of the allergen (for example, (a) in FIG. 3C) is then reduced to a thiol group by being donated an electron from the active fusion protein (for example, (b) in FIG. 3C). This inactivates the allergen.

Redox molecules are molecules that reduce redox enzymes. Redox molecules reduce redox enzymes by donating electrons to the redox enzymes. More specifically, redox molecules reduce redox enzymes by donating electrons donated by working electrode 1a to the redox enzymes. Redox molecules may donate electrons from working electrode 1a via electron mediators. The redox molecule is, for example, nicotinamide adenine dinucleotide phosphate (NADPH) or ferredoxin. The redox molecule may be nicotinamide adenine dinucleotide (NADH).

Electron mediators are redox substances that mediate electron transfer between the electrode and the redox proteins. Electron mediators donate electrons to redox molecules. More specifically, electron mediators donate electrons donated from working electrode 1a to redox molecules. The electron mediators may donate electrons directly to oxidized redox proteins. The electron mediator is not particularly limited as long as it enables electron transfer between the electrode and the redox proteins. The electron mediator may be selected according to the redox potential of the target molecule to which the electron mediator donates electrons. The electron mediator may be, for example, a compound with a bipyridine skeleton, a compound with a quinone skeleton, or a compound with a phenylenediamine skeleton. These compounds may be used alone, and, alternatively, a combination of two or more of these compounds may be used.

A compound with a bipyridine skeleton may be, for example, a compound with a 2,2'-bipyridine skeleton, a compound with a 2,4'-bipyridine skeleton, a compound with a 4,4'-bipyridine skeleton, or a derivative of these (for example, a 4,4'-bipyridinium derivative). A compound with a bipyridine skeleton may be a bipyridine compound with substituents on the bipyridine skeleton (i.e., a bipyridine derivative) or a bipyridine compound without substituents. Substituents include hydrogen, halogen, hydroxyl, nitro, carboxyl, carbonyl, amino, amide, or sulfonic acid groups, or alkyl, aryl, heteroaromatic alkyl, or phenyl groups substituted therewith. An aromatic ring may be formed between two adjacent substituents. The substituents are the same for a compound with a quinone skeleton and a compound with a phenylenediamine skeleton, as described below. When the electron mediator is a compound with a bipyridine skeleton, the electron mediator is, for example, 1,1'-dimethyl-4,4'-bipyridinium (methyl viologen), 1-methyl-1'-carboxylmethyl-4,4'-bipyridinium, 1,1'-dicarboxymethyl-4,4'-bipyridinium, 1-methyl-1'-aminoethyl-4,4'-bipyridinium, 1,1'-diaminoethyl-4,4'-bipyridinium, 1-methyl-1'-ethyl-4,4'-bipyridinium, 1-methyl-1'-propyl-4,4'-bipyridinium, 1-methyl-1'-butyl-4,4'-bipyridinium, 1-methyl-1'-pentylhexyl-4,4'-bipyridinium, 1-methyl-1'-hexyl-4,4'-bipyridinium, 1-methyl-1'-heptyl-4,4'-bipyridinium, 1-methyl-1'-octyl-4,4'-bipyridinium, 1-methyl-1'-nonyl-4,4'-bipyridinium, or 1-methyl-1'-decyl-4,4'-bipyridinium, or a compound in which the methyl group at position 1 of these compounds is replaced with an ethyl group.

A compound with a quinone skeleton may be, for example, a compound with a benzoquinone skeleton, a compound with a naphthoquinone skeleton, a compound with an anthraquinone skeleton, or a derivative of these. A compound with a quinone skeleton may or may not have substituents. As substituents have been discussed above, description here will be omitted. When the electron mediator is a compound with a quinone skeleton, the electron mediator may be, for example, methylbenzoquinone, dimethylbenzoquinone (such as 2,5-dimethyl-1,4-benzoquinone, 2,3-dimethyl-1,4-benzoquinone, and 2,6-dimethyl-1,4-benzoquinone), 2,3-dichloro-5,6-dicyano-1,4-benzoquinone, 2,3,5,6-tetramethyl-1,4- benzoquinone, 2,3,5,6-tetrachloro-1,4-benzoquinone (chloranil), ubiquinone (CoQ), pyrroloquinoline quinone (PQQ), 1,2-naphthoquinone-4-sulfonic acid, 2-methyl-1,4-naphthoquinone (vitamin K₃), 2-hydroxy-1,4-naphthoquinone, 1,2-dihydroxyanthraquinone (alizarin), 1,2,4-trihydroxyanthraquinone (purpurin), or 9,10-phenanthrenequinone. The electron mediator may be, for example, a benzenediol in which the ketone group of the benzoquinone skeleton is replaced with a hydroxyl group, or more specifically, hydroquinone in which the ketone group of 1,4-benzoquinone is replaced with a hydroxyl group (1,4-benzenediol), or resorcinol in which the ketone group of 1,3-benzoquinone is replaced with a hydroxyl group (1,3-benzenediol).

A compound with a phenylenediamine skeleton may or may not have substituents, for example. When the electron mediator is a compound with a phenylenediamine skeleton, the electron mediator may be, for example, p-phenylenediamine, 2,3,5,6-tetramethyl-1,4-phenylenediamine, N,N-dimethyl-p-phenylenediamine, N,N'-diphenyl-p - phenylenediamine (DPPD), N-isopropyl-N'-phenyl-p-phenylenediamine (IPPD), or N -(1,3-dimethylbutyl)-N'-phenyl-p-phenylenediamine (6PPD).

Referring again to FIG. 1, terminal 6a, terminal 6b, and terminal 6c that electrically connect working electrode 1a, reference electrode 2, and counter electrode 3 to power supply 20, respectively, are arranged on lid 5. Leads extend from each terminal, connecting the terminals to a battery. Working electrode 1a is connected to terminal 6a via lead 7a, reference electrode 2 is connected to terminal 6b via lead 7b, and counter electrode 3 is connected to terminal 6c via lead 7c.

### Power Supply

Power supply 20 applies voltage to an electrode (working electrode 1a). More specifically, power supply 20 applies voltage between working electrode 1a and counter electrode 3 of voltage applier 10a and controls the potential difference between working electrode 1a and reference electrode 2 to a predetermined value in accordance with a control signal output from controller 30. For example, power supply 20 may apply voltage so that the voltage applied to working electrode 1a is between -1.0 V and 0 V, inclusive, with reference electrode 2 as the reference (0 V). Here, reference electrode 2 is, for example, a silver/silver chloride electrode.

As illustrated in FIG. 2, power supply 20 includes, for example, obtainer 21, information processor 22, and voltage controller 23.

Obtainer 21 obtains a control signal output from controller 30. Obtainer 21 may also obtain measurement data such as the potential of each electrode in voltage applier 10a and the current value flowing in sample solution 9a.

Information processor 22 processes the information obtained by obtainer 21. For example, when information processor 22 obtains a control signal from obtainer 21, information processor 22 outputs the obtained control signal to voltage controller 23. When voltage controller 23 starts applying voltage to the electrodes in voltage applier 10a, information processor 22 obtains measurement data such as the potential of each electrode in voltage applier 10a and the current value flowing in sample solution 9a, which are obtained from obtainer 21, and derives the voltage to be applied to working electrode 1a based on the obtained data such that the potential difference between working electrode 1a and reference electrode 2 is maintained at a predetermined value. Information processor 22 outputs, to voltage controller 23, a control signal that controls the voltage of working electrode 1a with the derived voltage.

Voltage controller 23 applies voltage to each electrode of voltage applier 10a based on the control signal output from information processor 22.

Although FIG. 1 illustrates an example where power supply 20 and controller 30 are separate units, power supply 20 may include controller 30.

### Controller

Controller 30 processes information for controlling the application of voltage by power supply 20. Controller 30 is realized, for example, by a processor, a microcomputer, or dedicated circuitry. FIG. 1 illustrates an example where controller 30 is a computer.

Controller 30 includes, for example, obtainer 31, information processor 32, storage 33, and outputter 34.

Obtainer 31 obtains, for example, information related to instructions input by the user (hereinafter referred to as "instruction information"), and outputs the obtained instruction information to information processor 32.

Information processor 32 derives, for example, the conditions under which voltage is to be applied to each electrode of voltage applier 10a (also called voltage application conditions) based on the instruction information obtained by obtainer 31. The instruction information may be, for example, the type of allergen, the amount of sample solution 9a, the amount of time until completion of the process, the time of completion, or the degree of inactivation (reduction) (in percentage or a five-step display, for example).

Information processor 32 may output a control signal to outputter 34 to control voltage application under the conditions derived based on the instruction information, and, alternatively, may output a control signal to outputter 34 to control voltage application under voltage application conditions set in advance by the user.

Outputter 34 outputs the control signal obtained from information processor 32 to power supply 20.

Storage 33 stores data, such as the instruction information, obtained by obtainer 31 and computer programs (for example, an application program for controlling power supply 20) executed by controller 30.

### 3. Operation

Next, the operation of allergen inactivation device 100a according to Embodiment 1 will be described with reference to FIG. 1 through FIG. 4. FIG. 4 is a flowchart illustrating one example of the operation of allergen inactivation device 100a according to Embodiment 1.

First, the preparation process (not illustrated in the drawings) performed before operating allergen inactivation device 100a will be described. For example, the preparation process may be performed by the user. In the preparation process, first, sample solution 9a is prepared. The user introduces an allergen-containing sample into cell 4 of voltage applier 10a. Next, sample solution 9a is prepared by adding redox proteins, redox enzymes, redox molecules, and electron mediators to the sample in cell 4. The allergens present in sample solution 9a are inactivated by being reduced by the reduced redox proteins. The added redox proteins, redox enzymes, redox molecules, and electron mediators may all be in reduced form or a mixture of oxidized and reduced forms.

Next, the user inserts the electrodes into sample solution 9a and sets the electrodes in place. The electrodes are specifically working electrode 1a, reference electrode 2, and counter electrode 3. Working electrode 1a is connected to lead 7a extending from terminal 6a arranged on lid 5, reference electrode 2 is connected to lead 7b extending from terminal 6b arranged on lid 5, and counter electrode 3 is connected to lead 7c extending from terminal 6c arranged on lid 5.

Next, the user inputs, to allergen inactivation device 100a, information related to instructions (i.e., the instruction information), such as the type of allergen, the amount of sample solution 9a, the amount time of time until completion of the process, the completion time, or the degree of inactivation (reduction).

In the above preparation process, the user prepared sample solution 9a by adding, to the allergen-containing sample, redox proteins, redox enzymes, redox molecules, and electron mediators in reduced form only or in a mixture of reduced and oxidized forms, but oxidized redox proteins, oxidized redox enzymes, oxidized redox molecules, and oxidized electron mediators may also added. This reduces variation in inactivation efficiency because allergens present in sample solution 9a are inactivated after the voltage application is started in step S102.

In the above preparation process, sample solution 9a is prepared by introducing an allergen-containing sample and the like into cell 4, but a pre-prepared sample solution 9a may be introduced into cell 4.

Next, the operation of allergen inactivation device 100a will be described. After the instruction information is input by the user, controller 30 sets the conditions for applying voltage to each electrode of voltage applier 10a (step S101). In setting the conditions, controller 30 derives the voltage application conditions based on the input instruction information and outputs, to power supply 20, a control signal that controls the voltage application by power supply 20 under the derived voltage application conditions. In step S101, the user may select a program number associated with the voltage application conditions, and controller 30 may obtain the program number and set the voltage application conditions.

Next, power supply 20 obtains the control signal output from controller 30 and starts applying voltage to the electrodes in accordance with the obtained control signal (step S102). For example, power supply 20 applies a voltage between working electrode 1a and counter electrode 3 of voltage applier 10a to control the potential difference between working electrode 1a and reference electrode 2 to a predetermined value (for example, a value in the range between -1.0 V and 0 V, inclusive). In other words, power supply 20 applies voltage so that the voltage applied to working electrode 1a is between -1.0 V and 0 V, inclusive, with reference electrode 2 as the reference (0 V). Here, reference electrode 2 is, for example, a silver/silver chloride electrode. This causes electrons to be donated from working electrode 1a to oxidized redox proteins in sample solution 9a. As a result, the oxidized redox proteins are reduced to reduced redox proteins (step S103). The reduced redox proteins are then inactivated by reducing allergens in sample solution 9a (step S104). Step S103 and step S104 are repeated while voltage is applied to the electrodes from power supply 20. In step S103, the redox enzymes, redox molecules, and electron mediators in sample solution 9a are also reduced from their oxidized forms to their reduced forms.

Next, controller 30 determines whether processing under the set conditions is complete (step S105). The conditions set are, for example, the duration (time) of the voltage application or the number of times the voltage is applied (for example, pulsed voltage). If controller 30 determines that the processing under the set conditions is not complete (No in step S105), controller 30 causes power supply 20 to continue applying voltage (step S106). Steps S103 and S104 are then repeated until the next decision (step S105) is made. However, if controller 30 determines that processing under the set conditions is complete (Yes in step S105), controller 30 causes power supply 20 to end the application of voltage (step S107). This completes the inactivation of allergens in sample solution 9a.

Although the above preparation process is exemplified as performed by the user, the preparation process may be performed by allergen inactivation device 100a. In such cases, allergen inactivation device 100a may further include an introducer (not illustrated in the drawings), a collector (not illustrated in the drawings), an introduction port (not illustrated in the drawings), and an outlet port (not illustrated in the drawings). For example, the introducer may introduce the allergen-containing sample, redox proteins, redox enzymes, redox molecules, and electron mediators into cell 4 through an introduction port in cell 4. For example, the collector may collect the allergen-inactivated sample solution 9a out of cell 4 through an outlet port in cell 4.

### [Embodiment 2]

Next, Embodiment 2 will be described in detail with reference to FIG. 5 and FIG. 6. In Embodiment 2, description will focus on the points of difference from Embodiment 1. Description of content that overlaps with Embodiment 1 will be simplified or omitted.

### Allergen Inactivation Device

### 1. Overview

First, an overview of the allergen inactivation device according to Embodiment 2 will described with reference to FIG. 5. FIG. 5 illustrates one example of the configuration of allergen inactivation device 100b according to Embodiment 2.

Allergen inactivation device 100b differs from Embodiment 1 in that the redox proteins that reduce the allergens are immobilized on the electrode (in this case, working electrode 1b), so there is no need to prepare a sample solution. Allergen inactivation device 100b is a device that continuously inactivates allergens in a solution by repeatedly activating redox proteins that have lost their electrode reducing power, by donating electrons to the redox proteins that inactivate the allergens by reduction. Stated differently, allergen inactivation device 100b can directly inactivate allergens in the sample. This allows allergen inactivation device 100b according to Embodiment 2 to inactivate allergens more easily. Allergen inactivation device 100b can prevent, for example, redox proteins from being mixed into the allergen-containing sample.

### 2. Configuration

Next, the configuration of allergen inactivation device 100b according to Embodiment 2 will be described with reference to FIG. 5.

As illustrated in FIG. 5, allergen inactivation device 100b according to Embodiment 2 includes voltage applier 10b, power supply 20, and controller 30. Allergen inactivation device 100b differs from Embodiment 1 in that it includes working electrode 1b with redox proteins immobilized on the electrode surface thereof.

### Working Electrode

Working electrode 1b is an electrode for donating electrons to redox proteins by voltage application. The redox proteins are immobilized on working electrode 1b. Furthermore, in addition to the redox proteins, redox enzymes that donate electrons to the redox proteins may be immobilized on working electrode 1b, redox enzymes and redox molecules that donate electrons to the redox enzymes may be immobilized on working electrode 1b, and redox enzymes, redox molecules, and electron mediators that donate electrons to the redox molecules may be immobilized on working electrode 1b. Furthermore, in addition to the redox proteins, electron mediators may be immobilized on working electrode 1b. Hereinafter, an example in which redox proteins, redox enzymes, redox molecules, and electron mediators are immobilized on working electrode 1b will be described.

FIG. 6 is a schematic cross-sectional view taken at line VI-VI of working electrode 1b illustrated in FIG. 5. As illustrated in FIG. 6, working electrode 1b includes base electrode 18, and redox proteins 14, redox enzymes 15, redox molecules 16, and electron mediators 17 immobilized on base electrode 18. Base electrode 18 includes substrate 11 made of electrode material, conductive polymer 12, and conductive particles 13. Since redox proteins 14, redox enzymes 15, redox molecules 16, and electron mediators 17 (hereinafter also collectively referred to as "redox protein 14, etc.") have been mentioned above, repeated description will be omitted here.

Substrate 11 is made of, for example, a porous electrode material. From the viewpoint of strength, stiffness, and light weight, the conductive material may be, for example, carbon fiber, such as polyacrylonitrile (PAN)-based carbon fiber, pitch-based carbon fiber, or rayon-based carbon fiber. Among these, from the viewpoint of mechanical strength of the porous electrode material, the conductive material may be PAN-based carbon fiber. PAN-based carbon fiber may be, for example, a short-fiber randomly oriented mat formed of TORAYCA (registered trademark) yarn. One type of carbon fiber may be used, or several different types of carbon fiber may be used. Other known fibers, such as glass fiber, aramid fiber, polyethylene terephthalate fiber, vinylon fiber, polyester fiber, amide fiber, or ceramic fiber may also be used with the carbon fiber.

Conductive polymer 12 is not limited so long as it is a polymer having conductive properties, and may be, for example, polyacetylene, polythiophene, polyfluorene, polyethylenevinylene, polyphenylenevinylene, polypyrrole, or polyaniline. Conductive polymer 12 may contain a dopant.

Conductive particles 13 are not limited so long as they are particles that behave as good electrical conductors. For example, conductive particles 13 may be conductive polymer particles such as polyacetylene particles, polyaniline particles, polypyrrole particles, polythiophene particles, polyisothianaphthene particles, and polyethylene dioxithiophene particles, and may be carbon particles, carbon fiber particles, or metal particles. Among these, conductive particles 13 may be carbon or metal particles, as they exhibit high conductivity and stability.

Carbon particles may be carbon nanofibers, including carbon black, expanded graphite, scale graphite, graphite powder, graphite particles, graphene sheets, carbon milled fiber, carbon nanotubes, and vapor-phase grown carbon fiber (VGCF; registered trademark). Among these, carbon black and carbon milled fiber may be used as they exhibit high conductivity and are inexpensive. Carbon black may be furnace black, acetylene black, thermal black, channel black, or Ketjen black (registered trademark).

The metal particles are not particularly limited, but when carbon fiber is used as a reinforcing fiber, particles of platinum, gold, silver, copper, tin, nickel, titanium, cobalt, zinc, iron, chromium, aluminum, particles of alloys mainly composed of these metals, tin oxide, indium oxide, and indium tin oxide (ITO) are acceptable because they prevent corrosion due to potential difference with the carbon fiber.

The shape of conductive particles 13 is not particularly limited, and may be spherical, non-spherical, or porous particles. From the viewpoint of forming conductive bridges between carbon fiber layers, it is desirable to have a large aspect ratio.

The method of immobilizing redox proteins 14, etc., onto the surface of the electrode is not particularly limited; possible methods include, for example, chemically immobilizing redox proteins 14, etc., onto the electrode, indirectly immobilizing redox proteins 14, etc., onto the electrode using a conductive polymer or cross-linking agent, and immobilizing redox proteins 14, etc., onto the electrode via monolayer-forming molecules. In the example in FIG. 6, conductive polymers are used to immobilize redox proteins 14, etc., onto the electrode.

### 3. Operation

The operation of allergen inactivation device 100b according to Embodiment 2 is substantially the same as the operation of allergen inactivation device 100a according to Embodiment 1 illustrated in FIG. 4, so the flow of the implementation example will be omitted. Embodiment 2 differs from the operation of allergen inactivation device 100a according to Embodiment 1 in that it reduces oxidized redox proteins immobilized on working electrode 1b (specifically, step S103). Additionally, the preparation process does not require a process for preparing a sample solution. More specifically, the inventors discovered the method according to the present disclosure by causing allergen inactivation device 100b to repeatedly reduce oxidized redox proteins (for example, oxidized thioredoxin) to reduced redox proteins (for example, reduced thioredoxin). The inventors found that this makes it possible to efficiently inactivate allergens using a smaller amount of redox protein than is needed to inactivate all allergens.

### [Variation of Embodiment 2]

Embodiment 2 describes an example in which at least redox proteins are immobilized on working electrode 1b, but redox proteins do not need to be immobilized on working electrode 1b. For example, electron mediators that mediate the electron transfer between working electrode 1b and the redox proteins should be immobilized on the working electrode. Here, the sample solution contains allergens and redox proteins. This improves the inactivation efficiency of allergens since electron transfer efficiency is improved compared to when the redox proteins receive electrons directly from the working electrode.

### Implementation Examples

Hereinafter, implementation examples of the allergen inactivation method according to the present disclosure will be described in detail, but the present disclosure is not limited to the following implementation examples in any way.

In the following implementation examples, the rate of allergen degradation (digestibility) with and without the application of voltage to the allergen-containing sample solution was verified in each of the cases (1) and (2) described below. In the following, allergenic proteins with disulfide bonds (hereinafter simply referred to as allergenic proteins) were used as the allergens.

### (1) When Redox Proteins Are Not Immobilized on the Electrode Surface

When redox protein are not immobilized on the electrode surface, the redox proteins do not receive electrons directly from the electrode via interfacial electron transfer, but indirectly from the electrode via electron mediators, redox molecules, or redox enzymes. Hereafter, this is referred to as redox protein reduction by indirect electron transfer.

In this case, the redox proteins are included in the sample solution (hereafter referred to as Sample Solution I) along with the allergens.

### Comparative Example 1

In Comparative Example 1, Sample Solution I was allowed to stand overnight at a low temperature without voltage applied, and then digestive enzymes were added, and Sample Solution I was incubated under predetermined conditions.

### Preparation of Sample Solution I

Sample Solution I was prepared by dissolving, in phosphate buffered saline (PBS) at pH 7.4: the target molecules, i.e., allergens; oxidized redox proteins (i.e., inactive redox proteins); redox enzymes that reduce the oxidized redox proteins; redox molecules that activate the redox enzymes; and electron mediators that donate electrons to the oxidized redox molecules. The allergens are, for example, allergenic proteins with disulfide bonds. The redox enzyme and the redox protein used in Comparative Examples 1 and 2 and Implementation Examples 1 and 2 were NADPH-thioredoxin reductase and thioredoxin, and were prepared as described in PTL 1. NADPH was used as the redox molecule and methyl viologen was used as the electron mediator.

### Allergen Inactivation

After Sample Solution I was prepared, Sample Solution I was allowed to stand overnight at a low temperature (for example, the internal temperature of a refrigerator). Voltage was not applied to Sample Solution I.

### Allergen Degradation via Digestive Enzymes

After Sample Solution I was allowed to stand overnight, digestive enzymes were added to Sample Solution I, and Sample Solution I was incubated at 37°C for 2 hours. Incubated Sample Solution I and molecular weight markers were then electrophoresed by sodium dodecyl sulfate-Polyaclamide gel electrophoresis (SDS-PAGE) using the usual method. Next, the electrophoresed gel was stained, and the density of allergenic protein bands was quantified by image analysis. The electrophoresis images are illustrated in FIG. 7. FIG. 7 illustrates electrophoresis images after SDS-PAGE in Comparative Example 1 and Implementation Example 1. The electrophoresis images of the molecular weight markers are illustrated in (a) in FIG. 7. The electrophoresis images of Comparative Example 1 are illustrated in (b) in FIG. 7; the allergenic protein band was observed at the position surrounded by the dashed line. Although not illustrated in FIG. 7, electrophoresis of a sample solution containing only allergens (hereafter referred to as the control sample solution) was performed by SDS-PAGE, as an indicator of 100% retention.

### Calculating Rate of Allergen Degradation

The values indicating the concentration of the allergenic protein bands in the control sample solution and Sample Solution I of Comparative Example 1, respectively, were calculated. Then, the retention rate of allergenic proteins in Comparative Example 1 was calculated by proportional calculation, using the value corresponding to the control sample solution as 100% allergenic protein retention. As a result, in Comparative Example 1, the allergenic protein retention rate was 100%. Thus, in Comparative Example 1, the allergenic proteins in Sample Solution I were not degraded by the digestive enzymes (i.e., the degradation rate was 0%). The degradation rate calculation results are illustrated in FIG. 8. FIG. 8 is a graph illustrating the degradation rate of allergenic proteins after treatment with digestive enzymes in Comparative Example 1 and Implementation Example 1.

### Implementation Example 1

In Implementation Example 1, Sample Solution I was applied with a predetermined voltage overnight, at a low temperature, and then the digestive enzymes were added and Sample Solution I was incubated under predetermined conditions. Sample Solution I was prepared the same as in Comparative Example 1.

### Allergen Inactivation

In Implementation Example 1, a predetermined voltage was applied to Sample Solution I overnight, at a low temperature, using a three-electrode voltage-applying cell (for example, voltage applier 10a in FIG. 1) and a potentiostat (for example, power supply 20 in FIG. 1). Among the three electrodes, a glassy carbon electrode was used for working electrode 1a and an Ag/AgCl electrode was used for reference electrode 2. The predetermined voltage applied to Sample Solution I was controlled by the potentiostat so that the potential of working electrode 1a relative to reference electrode 2 was equal to the reduction potential of the electron mediator.

### Allergen Degradation via Digestive Enzymes

Next, Sample Solution I was collected after the voltage was applied, digestive enzymes were added to the collected Sample Solution I, and Sample Solution I was incubated at 37 °C for 2 hours. Incubated Sample Solution I and molecular weight markers were electrophoresed by SDS-PAGE, just as in Comparative Example 1. Staining of the gel after electrophoresis showed no bands of allergenic proteins. The electrophoresis images are illustrated in FIG. 7. The electrophoresis images of Implementation Example 1 are illustrated in (c) in FIG. 7; no allergenic protein bands were observed at the position surrounded by the dashed line.

### Calculating Rate of Allergen Degradation

Just like in Comparative Example 1, the values indicating the concentration of the allergenic protein bands in the control sample solution and Sample Solution I of Implementation Example 1 were calculated. Then, the retention rate of allergenic proteins in Implementation Example 1 was calculated by proportional calculation, using the value indicating density of the band of Comparative Example 1 as 100% allergenic protein retention. As a result, in Implementation Example 1, the retention rate of allergenic proteins in Sample Solution I was 0%.

Next, the degradation rate of allergenic proteins in Implementation Example 1 was calculated by subtracting the retention rate of Implementation Example 1 (0%) from the retention rate of Comparative Example 1 (100%). The degradation rate calculation results are illustrated in FIG. 8. As illustrated in FIG. 8, in Implementation Example 1, the degradation rate of allergenic proteins in Sample Solution I was 100%.

### (2) Immobilization of Redox Proteins on the Electrode Surface

When redox proteins are immobilized on the electrode surface, the redox proteins are reduced by indirect electron transfer, just as in (1) described above.

In such cases, redox proteins are not included in the sample solution (hereafter referred to as Sample Solution II), and only allergens are included as target molecules in Sample Solution II.

### Comparative Example 2

In Comparative Example 2, Sample Solution II was allowed to stand overnight at a low temperature without voltage applied, and then digestive enzymes were added, and Sample Solution II was incubated under predetermined conditions, just as in Comparative Example 1.

### Preparation of Sample Solution

Sample Solution II was prepared by dissolving target molecules, i.e., allergens in PBS at pH 7.4. The allergens are, for example, allergenic proteins with disulfide bonds.

### Production of the Electrode with Immobilized Redox Proteins

### 1. Making the Base Electrode Substrate

60 mg of conductive carbon black (for example, Ketjen black) was ground using an agate pestle. The ground conductive carbon black was mixed with 1800 µl of a N-methyl-2-pyrrolidone (NMP) solution while adding it gradually. In addition, 260 µl of a 10% (w/v) NMP solution of poly(4-vinylpyridine) (PVP) as a dispersion aid was added gradually while mixing. To this, 1800 µl of an NMP solution was added gradually while mixing. This was transferred to a 25 ml container and the conductive carbon black was dispersed in the NMP solution by sonication to obtain a carbon slurry. The resulting carbon slurry was impregnated into a 1 cm diameter carbon fiber mat (TORAYCA mat) to obtain prepreg. The base electrode substrate was obtained by drying the prepreg at 90 °C for 3 hours.

### 2. Immobilization of Redox Proteins

A redox protein solution containing redox proteins, redox enzymes, redox molecules, and electron mediators was prepared. Next, 25 µl of this redox protein solution was mixed with 4.1 µl of a 20% (w/v) poly L-lysine solution, 4.4 µl of a 2.5% glutaraldehyde solution, 7.2 µl of a 10 mM Tris-HCl buffer, 1.5 µl of a 50 mg/ml BSA (bovine serum albumin) solution, and 12.8 µl of distilled water. After the base electrode substrate was impregnated with this immobilization solution, the base electrode substrate was allowed to stand at 4 °C for at least 8 hours to dry. This resulted in an electrode (working electrode 1b in FIG. 5) with redox proteins, redox enzymes, redox molecules, and electron mediators immobilized on the surface of the base electrode substrate. Note that the redox proteins immobilized on the electrode were oxidized (inactive) redox proteins.

### Inactivation of Allergens and Degradation of Allergens by Digestive Enzymes

The process was the same as in Comparative Example 1, except that Sample Solution II was used instead of Sample Solution I. The electrophoresis results are illustrated in FIG. 9. FIG. 9 illustrates electrophoresis images after SDS-PAGE in Comparative Example 2 and Implementation Example 2. The electrophoresis images of the molecular weight markers are illustrated in (a) in FIG. 9. The electrophoresis images of Comparative Example 2 are illustrated in (d) in FIG. 9; the allergenic protein band was observed at the position surrounded by the dashed line. Although not illustrated in FIG. 9, electrophoresis of a sample solution containing only allergens (hereafter referred to as the control sample solution) was performed by SDS-PAGE, as an indicator of 100% retention.

### Calculating Rate of Allergen Degradation

Just as in Comparative Example 1, the values indicating the concentration of the allergenic protein bands in the control sample solution and Sample Solution II of Comparative Example 2, respectively, were calculated. Then, the retention rate of allergenic proteins in Comparative Example 2 was calculated by proportional calculation, using the value corresponding to the control sample solution as 100% allergenic protein retention. As a result, in Comparative Example 2, the retention rate of allergenic proteins was 100%. Thus, in Comparative Example 2, the allergenic proteins in Sample Solution I were not degraded by the digestive enzymes (i.e., the degradation rate was 0%). The degradation rate calculation results are illustrated in FIG. 10. FIG. 10 is a graph illustrating the degradation rate of allergenic proteins after treatment with digestive enzymes.

### Implementation Example 2

Implementation Example 2 was conducted in the same manner as Implementation Example 1, except that Sample Solution II was used instead of Sample Solution I and that an electrode (working electrode 1b in FIG. 5) with immobilized redox proteins, etc., was used.

### Allergen Inactivation

In Implementation Example 2, a predetermined voltage was applied to Sample Solution II overnight, at a low temperature, using a three-electrode voltage-applying cell (for example, voltage applier 10b in FIG. 5) and a potentiostat (for example, power supply 20 in FIG. 5). Among the three electrodes, an electrode with immobilized redox proteins, etc., described above was used for working electrode 1b, and an Ag/AgCl electrode was used for the reference electrode. The predetermined voltage applied to Sample Solution II was controlled by the potentiostat so that the potential of working electrode 1b relative to reference electrode 2 was equal to the reduction potential of the electron mediator.

### Allergen Degradation via Digestive Enzymes

Next, Sample Solution II was collected after the voltage was applied, and the same procedure as in Implementation Example 1 was used for enzymatic treatment of Sample Solution II with digestive enzymes and electrophoresis by SDS-PAGE. The electrophoresis images are illustrated in FIG. 9. The electrophoresis images of Implementation Example 2 are illustrated in (e) in FIG. 9; an allergenic protein band was observed at the position surrounded by the dashed line.

### Calculating Rate of Allergen Degradation

Just like in Comparative Example 2, the values indicating the concentration of the allergenic protein bands in the control sample solution and Sample Solution II of Implementation Example 2 were calculated. Then, the retention rate of allergenic proteins in Implementation Example 2 was calculated by proportional calculation, using the value indicating density of the band of Comparative Example 2 as 100% allergenic protein retention. As a result, in Implementation Example 2, the retention rate of allergenic proteins in Sample Solution II was 64%.

Next, the degradation rate of allergenic proteins in Implementation Example 2 was calculated by subtracting the retention rate of Implementation Example 2 (64%) from the retention rate of Comparative Example 2 (100%). The degradation rate calculation results are illustrated in FIG. 10. As illustrated in FIG. 10, in Implementation Example 2, the degradation rate of allergenic proteins in Sample Solution II was 36%.

### Observations

The results of Comparative Examples 1 and 2 suggest that when no voltage is applied to the sample solution, oxidized redox proteins are not reduced to reduced redox proteins. Stated differently, this suggests that if no voltage is applied to the sample solution, the disulfide bonds of allergenic proteins (hereafter referred to as allergens) will not be reduced. It is therefore believed that the allergens were not degraded by the digestive enzymes because enzymatic treatment with digestive enzymes was performed on the sample solution while the disulfide bonds of the allergens were not reduced (i.e., not cleaved).

However, the results of Implementation Examples 1 and 2 suggest that oxidized redox proteins are reduced to reduced redox proteins when voltage is applied to the sample solution. Stated differently, this suggests that oxidized redox proteins are repeatedly reduced to reduced redox proteins by indirect electron transfer from the electrode when voltage is applied to the sample solution. The higher degradation rates by digestive enzymes in Implementation Examples 1 and 2 compared to Comparison Examples 1 and 2 suggest the possibility that with the application of voltage to the sample solution, the oxidized redox proteins are repeatedly reduced via the mechanism described above, and the reduced redox proteins are continuously cleaving the disulfide bonds of the allergen. Therefore, it is believed that with the allergen inactivation method according to the present disclosure, by applying voltage to the sample solution, electrons are donated from the electrode to the oxidized redox proteins, and the oxidized redox proteins are reduced to reduced redox proteins. This suggests that the allergen inactivation method according to present disclosure can efficiently inactivate allergens present in the sample solution because the oxidized redox proteins in the sample solution are repeatedly reduced to reduced redox proteins.

Based on the results of both Implementation Examples 1 and 2, the lower degradation rate in Implementation Example 2 despite the inactivation process being performed under the same voltage application conditions may be due to the fact that the amount of redox protein immobilized on the electrode in Implementation Example 2 was less than that of the redox protein added in Sample Solution I of Implementation Example 1. Furthermore, when redox proteins are immobilized on the electrode, electron transfer occurs near the interface of the electrode, which may also be one reason why the allergens are not easily reduced unless they are close to the interface of the electrode. Although the data is not shown, a study of agitation conditions for the sample solution showed that constant agitation decreased the rate of allergen degradation, indicating the need to consider the balance between inactivation of allergens near the interface of the electrode and reduction of oxidized redox proteins.

Regarding the increased degradation rate of the allergenic proteins by the digestive enzymes, it is believed that the disulfide bonds of the allergenic proteins in the sample solution are reduced by reduced redox proteins, which makes it easier for the digestive enzymes to act on the allergenic proteins. More specifically, the increased degradation rate of the allergenic proteins by the digestive enzymes is believed to be due to an increase in the degree of freedom (i.e., fluctuation) of the conformational structure of the allergenic proteins due to the cleavage of the disulfide bonds that connect the secondary structures of the allergenic proteins by reduction.

It is also conceivable that the reduction in allergenicity of the allergenic proteins is due to the increased fluctuation of the conformational structure of the allergenic proteins making it difficult for IgE antibodies, for example, to identify the specific binding sites of the allergenic proteins.

Therefore, with the allergen inactivation method according to the present disclosure, it is believed that the disulfide bonds of the allergens can be efficiently reduced with a small amount of redox protein because the redox proteins can be repeatedly activated (i.e., reduced) by applying voltage to the sample solution. With the allergen inactivation method according to the present disclosure, it is believed that the degradability of the allergenic protein by digestive enzymes is improved in addition to the allergenicity of the allergenic protein being reduced because the fluctuation of the conformational structure of the allergenic protein is increased by efficiently reducing (cleaving) the disulfide bonds in the allergens. Therefore, the allergen inactivation method according to the present disclosure is believed to efficiently inactivate allergens.

### (3) When the Number of Component Configurations in Sample Solution Is Changed without Electrode Immobilization

Next, the allergens were inactivated by varying the number of components in the sample solution.

### Implementation Example 3

Implementation Example 3 was conducted in the same manner as Implementation Example 1, except that Sample Solution III containing electron mediators, redox enzymes, and redox proteins was used instead of Sample Solution I. However, the redox enzymes and redox proteins used in Implementation Example 3 are ferredoxin-thioredoxin reductase and thioredoxin, and were prepared using the method disclosed in Non-patent Literature (NPL) 1 (Keisuke Yoshida et al., "Distinct electron transfer from ferredoxin-thioredoxin reductase to multiple thioredoxin isoforms in chloroplasts", Biochemical Journal, Portland Press, 2017, Vol. 474 (Pt. 8), pp. 1347-1360). The electrophoresis results are illustrated in FIG. 11. In FIG. 11, (a) and (d) are molecular weight markers, and (e) is a control sample (no treatment). As illustrated in (b) in FIG. 11, in Implementation Example 3, the retention rate of allergenic proteins in Sample Solution III was 55%.

The degradation rate of allergenic proteins in Implementation Example 3 was then calculated. The degradation rate calculation results are illustrated in FIG. 12. As illustrated in (b) in FIG. 12, in Implementation Example 3, the degradation rate of allergenic proteins in Sample Solution III was 45%.

### Implementation Example 4

Implementation Example 4 was conducted in the same manner as Implementation Example 1, except that Sample Solution IV, which contains electron mediators and fusion proteins of redox enzymes and redox proteins, was used instead of Sample Solution I. Thioredoxin reductase and thioredoxin fusion proteins were prepared and used as fusion proteins. The electrophoresis results are illustrated in FIG. 11. As illustrated in (c) FIG. 11, the retention rate of allergenic proteins in Sample Solution IV was 42%.

The degradation rate of allergenic proteins in Implementation Example 4 was then calculated. The degradation calculation rate results are illustrated in FIG. 12. As illustrated in (c) in FIG. 12, in Implementation Example 4, the degradation rate of allergenic proteins in Sample Solution IV was 58%.

### Observations

The results of Implementation Examples 3 and 4 show that when three molecules other than the redox molecules (the electron mediator, the redox enzyme, and the redox protein) or two molecules (the electron mediator and the redox enzyme-redox protein complex protein) are present in the sample solution, the retention rate of allergenic proteins decreases to about 50%. This confirms that the allergen inactivation effect is achieved regardless of the number of components involved in electron transfer.

Although the allergen inactivation method and the allergen inactivation device according to the present disclosure have been described above based on embodiments, the present disclosure is not limited to these embodiments. Various modifications to the above embodiments that may be conceived by those skilled in the art, as well as embodiments resulting from arbitrary combinations of elements from different embodiments that do not depart from the essence of the present disclosure are included the present disclosure.

Although the above embodiments describe an example of efficient inactivation of allergens using electrical energy by controlling the application of voltage, the present disclosure is not limited to this example. For example, in Embodiment 2, sample 9b may be agitated to increase the reactivity of the redox proteins immobilized on working electrode 1b and the allergens in sample 9b. In other words, in addition to voltage application conditions, controller 30 of allergen inactivation device 100b may derive agitation conditions such as agitation speed, agitation time, and agitation interval, and output a control signal related to voltage control and a control signal related to agitation control. In such cases, allergen inactivation device 100b may include an agitator that agitates sample 9b in cell 4 of voltage applier 10b. The agitator may include an agitator blade that is attachable to and removable from lid 5, a motor that rotates the agitator blade, and a controller that controls the movement of the motor.

### [Industrial Applicability]

The present disclosure can provide an allergen inactivation method that can efficiently inactivate allergens derived from food, drugs, pollen, animals, etc., and a device for implementing this method.

### [Reference Signs List]

- 1a,: 1bworking electrode
- 2: reference electrode
- 3: counter electrode
- 4: cell
- 5: lid
- 6a, 6b, 6c: terminal
- 7a, 7b, 7c: lead
- 9a: sample solution
- 9b: sample
- 10a, 10b: voltage applier
- 11: substrate
- 12: conductive polymer
- 13: conductive particle
- 14: redox protein
- 15: redox enzyme
- 16: redox molecule
- 17: electron mediator
- 18: base electrode
- 20: power supply
- 21: obtainer
- 22: information processor
- 23: voltage controller
- 24: outputter
- 30: controller
- 31: obtainer
- 32: information processor
- 33: storage
- 34: outputter
- 100a, 100b: allergen inactivation device

## Claims

1. An allergen inactivation method comprising:
inactivating an allergen present in a reaction system by reduction via a reduced redox protein; and
reducing an oxidized redox protein produced by oxidation of the reduced redox protein in the inactivating to the reduced redox protein by donating an electron from an electrode connected to an external power supply outside the reaction system to the oxidized redox protein.

2. The allergen inactivation method according to claim 1, wherein
in the reducing, an electron is donated from the electrode to a redox enzyme, and an electron is donated from the redox enzyme to the oxidized redox protein.

3. The allergen inactivation method according to claim 1, wherein
in the reducing, an electron is donated from the electrode to a redox molecule, an electron is donated from the redox molecule to a redox enzyme, and an electron is donated from the redox enzyme to the oxidized redox protein.

4. The allergen inactivation method according to claim 1, wherein
in the reducing, an electron is donated from the electrode to an electron mediator, an electron is donated from the electrode mediator to a redox molecule, an electron is donated from the redox molecule to a redox enzyme, and an electron is donated from the redox enzyme to the oxidized redox protein.

5. The allergen inactivation method according to any one of claims 1 to 4, wherein
the allergen includes a disulfide bond.

6. The allergen inactivation method according to any one of claims 1 to 5, wherein
the redox protein is thioredoxin, glutathione, a protein with at least one thioredoxin-like domain, or a protein with at least one glutathione-like motif.

7. The allergen inactivation method according to any one of claims 2 to 4, wherein
the redox enzyme is: (i) an enzyme that catalyzes reduction of oxidized thioredoxin, including a nicotinamide adenine dinucleotide phosphate (NADPH)-thioredoxin reductase or a ferredoxin-thioredoxin reductase; or (ii) an enzyme that catalyzes reduction of oxidized glutathione, including a glutathione reductase.

8. The allergen inactivation method according to claim 3 or 4, wherein
the redox molecule is nicotinamide adenine dinucleotide phosphate or ferredoxin.

9. The allergen inactivation method according to claim 4, wherein
the electron mediator is a compound with a bipyridine skeleton.

10. The allergen inactivation method according to any one of claims 1 to 9, wherein
a reaction temperature in the reaction system is greater than or equal to 4 °C and less than 60 °C.

11. The allergen inactivation method according to any one of claims 1 to 10, wherein
a voltage applied to the electrode by the external power supply is between -1.0 V and 0 V, inclusive.

12. An allergen inactivation device comprising:
an electrode for donating an electron to a redox protein that inactivates an allergen by reduction by application of voltage;
a power supply that applies voltage to the electrode; and
a controller that controls application of voltage by the power supply.

13. The allergen inactivation device according to claim 12, wherein
the redox protein is immobilized on the electrode.

14. The allergen inactivation device according to claim 12, wherein
a redox enzyme that donates an electron to the redox protein is further immobilized on the electrode.

15. The allergen inactivation device according to claim 14, wherein
a redox molecule that donates an electron to the redox enzyme is further immobilized on the electrode.

16. The allergen inactivation device according to claim 15, wherein
an electron mediator that donates an electron to the redox molecule is further immobilized on the electrode.

17. The allergen inactivation device according to claim 12 or 13, wherein
an electron mediator that mediates electron transfer between the electrode and the redox protein is immobilized on the electrode.
